# EUROPÄISCHE PATENTSCHRIFT

(11) **EP 1 427 704 B1**
(45) Veröffentlichungstag und Bekanntmachung des Hinweises auf die Patenterteilung: **09.02.2005**
(21) Anmeldenummer: 02760268.9
(22) Anmeldetag: 25.07.2002
(51) Int. Cl.: C07D 213/80, C07D 213/82, C07D 211/76, C07D 211/86, A01N 43/40, C07C 229/30, C07C 225/14, C07C 69/54

(54) **VERFAHREN ZUR HERSTELLUNG VON 4-HALOALKYLNICOTINSÄUREESTERN**
METHOD FOR PRODUCING 4-HALOALKYLNICOTINIC ACID ESTERS
PROCEDE DE PRODUCTION D'ESTERS D'ACIDE 4-HALOALKYLNICOTINIQUE

(30) Priorität: 11.09.2001 DE 10144410
(43) Veröffentlichungstag der Anmeldung: 16.06.2004
(73) Patentinhaber: Bayer CropScience GmbH, 65929 Frankfurt/Main (DE)
(72) Erfinder: PAZENOK, Sergiy, 65779 Kelkheim (DE)
(86) Internationale Anmeldenummer: PCT/EP2002/008275
(87) Internationale Veröffentlichungsnummer: WO 2003/022818

(56) Entgegenhaltungen:
- EP-A- 0 580 374
- EP-A- 0 744 400
- OKADA E ET AL: "A FACILE AND CONVENIENT SYNTHETIC METHOD FOR 3-TRIFLUOROACETYL-PYRROLES" HETEROCYCLES, XX, XX, Bd. 34, Nr. 7, 1992, Seiten 1435-1441, XP000196321 ISSN: 0385-5414
- OKADA E ET AL: "A FACILE AND CONVENIENT SYNTHETIC METHOD FOR N-BETA-TRIFLUOROACETYLVINYL AMINO ACID ESTERS, ALPHA-AMINOACETOPHENONES AND AMINOACETONITRILES AS POTENTIALLY USEFUL PRECURSORS OF FLUORINE-CONTAINING PYRROLES" SYNTHESIS, GEORG THIEME VERLAG. STUTTGART, DE, 1. Juni 1992 (1992-06-01), Seiten 533-535, XP000196319 ISSN: 0039-7881

## Beschreibung

Die Erfindung betrifft ein Verfahren zur Herstellung von 4-Haloalkyl-3-pyridincarbonsäureestern (4-Haloalkylnicotinsäureestern).

4-Haloalkylnicotinsäureamide sind nützliche Ausgangsstoffe zur Herstellung von Schädlingsbekämpfungsmitteln, wie sie beispielsweise in der WO-A 98/57 969, EP-A 0 580 374 und DE-A 100 14 006 beschrieben sind.

Die Herstellung dieser Verbindungen kann in zwei Stufen aus 4-Haloalkylnicotinsäuren erfolgen, deren Synthese durch die Ringschluß von 3-(4,4,4-Trifluor-3-oxo-1-butenylamino)-2-methylacrylat mit NaOMe beispielsweise in EP-A 0 744 400 beschrieben ist.

Es wurde nun überraschend ein einfaches Verfahren zur Herstellung von 4-Haloalkylnicotinsäuremethylestern (I) gefunden, aus dem sich 4-Haloalkylnicotinsäureamid in nur einem Schritt durch Ammonolyse erhalten läßt

Gegenstand der Erfindung ist daher ein Verfahren zur Herstellung von 4-Haloalkylnicotinsäureestern (I), 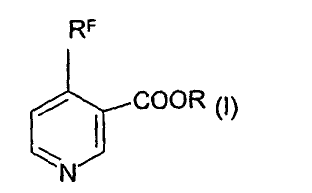 worin
- R^{F}: (C₁-C₄)-Haloalkyl und
- R: (C₁-C₆)-Alkyl bedeutet;
wobei man
a) ein Aminoketon der Formel (II),

   R^{F}-C(O)-CH=CH-NH₂ (II)

   Worin R^{F} die oben genannten Bedeutungen hat, in einer Kondensationsreaktion unter vermindertem Druck mit einer Verbindung der Formel (III) bis (V),

   (R¹Z)CH=CH-COOR (III),

   (R¹Z)₂CH-CH₂-COOR (IV),

   HC≡C-COOR (V),

   wobei R, R¹ glaich oder verschieden (C₁-C₆)-Alkyl und Z, gleich oder verschieden, O, S, NR¹ oder C(O)O bedeutet,
   zu einer Verbindung der Formel (VI) und/oder (VII) umsetzt,

   R^{F}-C(O)-CH=CH-NH-CH=CH-COOR (VI)

   R^{F}-C(O)-CH=CH-NH-CH(ZR¹)-CH₂-COOR (VII)

   worin R^{F}, R, R¹ und Z die oben angegebenen Bedeutungen haben,
   und das Reaktionsprodukt
b) einer Ringschlußreaktion unterwirft.

Überraschenderweise führt das erfindungsgemäße Verfahren zu 4-(Haloalkyl)-nicotinsäureestern, im Gegensatz z.B. zu dem in EP-A 0 744 400 beschriebenen, wo bei der Ringschlußreaktion die Säure oder ein Salz entsteht.

Weiterhin Gegenstand der Erfindung ist ein Verfahren zur Herstellung von Verbindungen der Formel (VI) und/oder VII), wobei man eine Verbindung der Formel (II),

R^{F}-C(O)-CH=CH-NH₂ (II)

in einer Kondensationsreaktion unter vermindertem Druck mit einer Verbindung der Formel (III) bis (V)

(R¹Z)CH=CH-COOR (III)

(R¹Z)₂C-CH₂-COOR (IV)

HC≡C-COOR (V)

umsetzt, wobei die Symbole die oben angegebenen Bedeutungen haben.

Weiterhin Gegenstand der Erfindung ist ein Verfahren zur Herstellung von 4-Haloalkylnicotinsäureamiden (VIII), 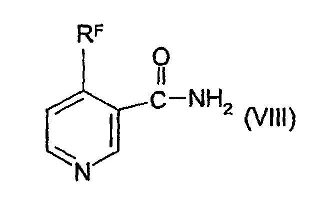 wobei man einen gemäß obigem Prozeß erhaltenen 4-Haloalkylnicotinsäureester (I) 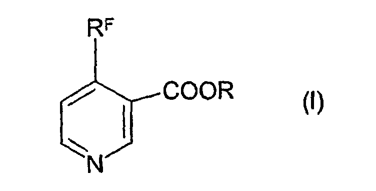 mit NH₃ umsetzt.

Ein besonderer ökonomischer Vorteil gegenüber der bekannten Synthese aus der Säure liegt darin, daß nach dem erfindungsgemäßen Verfahren kein aktiviertes Säurederivat, wie z.B. ein Säurechlorid, erforderlich ist.

Verbindungen der Formel (II), wie 4-Amino-1,1,1-trifluor-3-buten-2-on, sind bekannt und können beispielsweise wie in der EP-A 0 744 400 beschrieben hergestellt werden, indem ein Säurehalogenid der Formel (IX),

R^{F}-COX (IX)

worin X ein Halogenatom bedeutet,
mit einer Verbindung der Formel (X),

CH₂=CHOR² (X)

worin R² eine Alkylgruppe mit vorzugsweise 1 bis 6 C-Atomen bedeutet,
zu einer Verbindung der Formel (XI) umsetzt,

CF₃-C(O)-CH=CH(OR²) (XI)

aus der durch Reaktion mit Ammoniak Verbindung (II) erhalten wird.

Verbindungen der Formel (III) bis (V) sind bekannt. Sie sind kommerziell erhältlich oder können nach bekannten, dem Fachmann geläufigen Methoden, wie sie z.B. in J. Chem. Soc; 1969, 406-408; Bull. Soc. Chim. Fr. 1948, 594 und J. Org. Chem; 29, 1964,1800-1808 beschrieben sind, hergestellt werden.

R, R¹, R² sind gleich oder verschieden vorzugsweise eine lineare oder verzweigte Alkylgruppe mit 1 bis 6, vorzugsweise 1 bis 4, Kohlenstoffatomen, beispielsweise Methyl, Ethyl, n-Propyl, iso-Propyl, n-Butyl oder tert.-Butyl, bevorzugt sind Methyl und Ethyl, besonders bevorzugt ist Methyl.

R^{F} ist (C₁-C₄)-Haloalkyl, wobei Halo, F, Cl, Br und I, vorzugsweise F und Cl bedeutet. Bevorzugt sind CH₂F, CFCl₂, CF₂Cl, CF₃, C₂F₅ und C₃F₇, besonders bevorzugt CF₂Cl, C₂F₅ und CF₃, insbesondere CF₃.

Besonders bevorzugt ist eine Verbindung der Formel (I) mit R^{F} = CF₃ und R = Methyl.

Erfindungsgemäß wird Verbindung (II) in einer Kondensationsreaktion mit einer oder mehreren Verbindungen der Formeln (III) bis (V) zu Verbindung (VI) und/oder (VII) umgesetzt.

Die Kondensation von Verbindung (II) mit einer oder mehreren Verbindungen (III) bis (V) und die anschließende Ringschlußreaktion sind beispielhaft im folgenden Schema dargestellt:

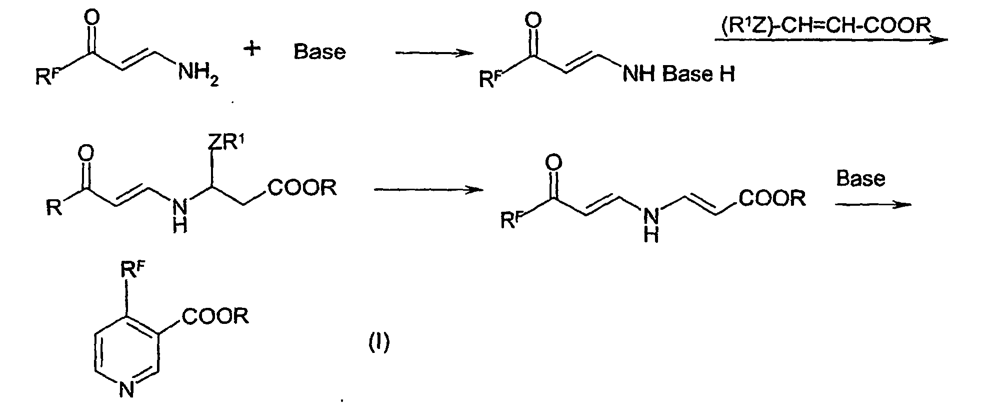

Die Reaktion wird erfindungsgemäß unter vermindertem Druck, bevorzugt bei einem Druck im Bereich von 5-150 mbar, besonders bevorzugt 10-100 mbar, insbesondere 20 bis 80 mbar, durchgeführt. Gleichzeitig destilliert man vorzugsweise die leichtsiedene Produkte von Reaktionsgemisch ab und ermöglicht dabei eine komplette Umsetzung beider Edukte. Der verminderte Druck wird vorteilhaft so gewählt, daß der Siedepunkt des abgespaltenen Verbindung R¹ZH, wie z.B. CH₃OH, EtOH, BuOH, unterhalb, vorzugsweise 50 bis 10°C unterhalb der Reaktionstemperatur liegt und der Siedepunkt des Lösemittels oberhalb, vorzugsweise 50 bis 80°C oberhalb der Reaktionstemperatur liegt.

Das Verhältnis der beiden Komponenten (II) und (III) bis (V) kann bei den Umsetzungen je nach eingesetzter Verbindung und weiteren Reaktionsbedingungen in weitem Umfang variieren.

Üblicherweise beträgt das Molverhältnis der Komponenten (II) : (III) bis (V) 1,0 -1,2 : 1, vorzugsweise 1,02 - 1,06 : 1.

Die Reaktionstemperatur kann je nach eingesetzter Verbindung und den sonstigen Reaktionsbedingungen in weiten Grenzen variieren. Im allgemeinen liegt die Reaktionstemperatur im Bereich von -20°C bis +100°C, vorzugsweise 0°C bis + 30°C und die Reaktionszeit beträgt üblicherweise 0,5 bis 12 h, vorzugsweise von 1 bis 6 h. Die Reaktionsbedingungen können variieren, je nachdem , welche Verbindung der Formel (III) bis (V) eingesetzt wird.

Als Base eignen sich beispielsweise Alkalimetallhydride, wie NaH oder KH, Alkyllithiumverbindungen, wie n-Butyllithium, oder t-Butyllithium, Alkalimetalle, wie Natrium oder Kalium, Alkoholate, wie Na-Methanolat, Na-Ethanolat, K-Methanolat oder K-t-Butanolat. Bevorzugt sind Alkoholate und Alkalimetallhydride, besonders bevorzugt sind: NaH; NaOMe, NaOtBut und KOtBut. Für eine großtechnische Anwendung sind NaOMe, NaOtBut und KOtBut bevorzugt. Die Basen können einzeln oder in Mischung eingesetzt werden. Die Menge der eingesetzten Base kann in weiten Grenzen variieren, je nachdem was für eine Verbindung der Formel (III) eingesetzt wird, ob und in welchem Lösungsmittel gearbeitet wird sowie den weiteren Reaktionsbedingungen. Im allgemeinen werden 1,0 bis 1,2 Äquivalente Base, vorzugsweise 1,02 bis 1,06 Äquivalente Base, pro Mol Verbindung der Formel (III) eingesetzt.

Die Reaktion wird vorzugsweise in einem Lösungsmittel durchgeführt. Dabei können die Komponenten (III) und (IV) beide in dem Lösungsmittel vorgelegt und diese Lösungen zusammen zur Reaktion gebracht werden, oder eine der Komponenten wird in dem Lösungsmittel vorgelegt, und die andere Komponente wird zugegeben, oder die Base wird zu einer Lösung der beiden Komponenten gegeben.

Als Lösungsmittel bevorzugt sind polare, aprotische Lösungsmittel, wie N,N-Dimethylformamid, N-Methylpyrollidon (NMP), Sulfolane oder Acetonitril und Kohlenwasserstoffe. Polare aprotische Lösungsmittel sind bevorzugt, besonders bevorzugt sind N,N-Dimethylformamid (DMF) und NMP. Es können auch Mischungen der genannten Lösungsmittel eingesetzt werden.

Die Menge des eingesetzten Lösungsmittels kann in weiten Grenzen variieren und hängt beispielsweise davon ab, ob und welche Base zugesetzt wird. Im allgemeinen beträgt die Menge des verwendeten Lösungsmittels 1 bis 30, vorzugsweise 4 bis 15 Gewichtsteile pro Gewichtsteil der Verbindung (III).

Die Herstellung von Verbindungen der Formel (Ia) durch Umsetzung einer Verbindung der Formel (II) mit einer Verbindung der Formel (IV) erfolgt in zwei Stufen, wobei zuerst unter Abspaltung der Gruppe (R¹Z) die Verbindung der Formel (Ib) gebildet wird und dann in einer zweiten Stufe ein weiteres Molekül R¹ZH abgespalten wird, was zu der Verbindung der Formel (la) führt.

### 1. Stufe

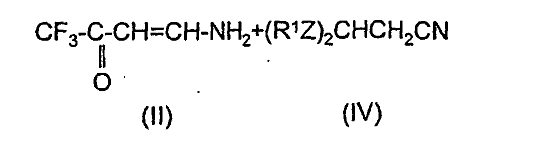

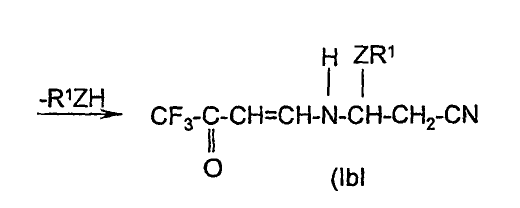

### 2.Stufe

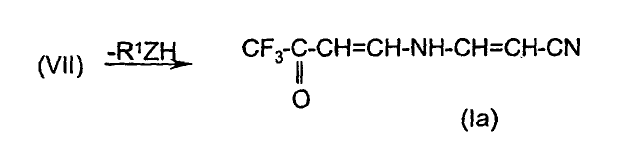

In allen Reaktionen können statt der reinen Verbindungen auch die Salze eingesetzt oder je nach Reaktionsführung erhalten werden.

Wird die Kondensationsreaktion in Gegenwart einer Base ausgeführt, die ein Alkalimetall enthält, bilden die Verbindungen (Ia) und (Ib) Alkalisalze. In solchen Fällen schließt an die Kondensationsreaktion ein Neutralisierungsschritt mit einer Säure an. Die Aufarbeitung erfolgt nach bekannten, dem Fachmann geläufigen Methoden, wie Filtration, Waschen und Trocknen.

In einer bevorzugten Ausführungsform wird das erfindungsgemäße Verfahren kontinuierlich durchgeführt, wobei beispielsweise eine Mischung der Verbindungen (II), (III) und/oder (IV) und Base in einem Lösungsmittel unter vermindertem Druck durch einen erhitzten Rohrreaktor geleitet wird.

Die Ringschlußreaktion der Verbindungen (VI) und/oder (VII) zur Verbindung (I) erfolgt vorteilhaft in einem Lösungsmittel. Bevorzugt sind Alkohole, besonders bevorzugt primäre (C₁-C₆)-Akohole, ganz besonders bevorzugt sind Methanol und Ethanol, insbesondere Methanol. Es können auch Mischungen der genannten Lösungsmittel eingesetzt werden.

Die Verbindungen (VI) und/oder (VII) können dabei in dem Lösungsmittel vorgelegt werden, oder das Lösungsmittel wird der Reaktionsmischung zugegeben.

Die Menge des eingesetzten Lösungsmittels für die Ringschlußreaktion kann je nach Ausgangsverbindung und Reaktionsbedingungen in weiten Grenzen variieren. Im allgemeinen beträgt sie 1 bis 30, vorzugsweise 4 bis 15 Gewichtsteile pro Gewichtsteil Verbindung (VI) und/oder (VII).

Die Ringschlußreaktion der Verbindungen (VI) und/oder (VII) erfolgt vorteilhaft in einem Alkohol als Lösungsmittel und in Gegenwart einer vorzugsweise schwachen Base zu den Zwischenprodukten (XII) und/oder (XIII). Bei anschließendem Ansäuern wird Verbindung (I) gebildet:

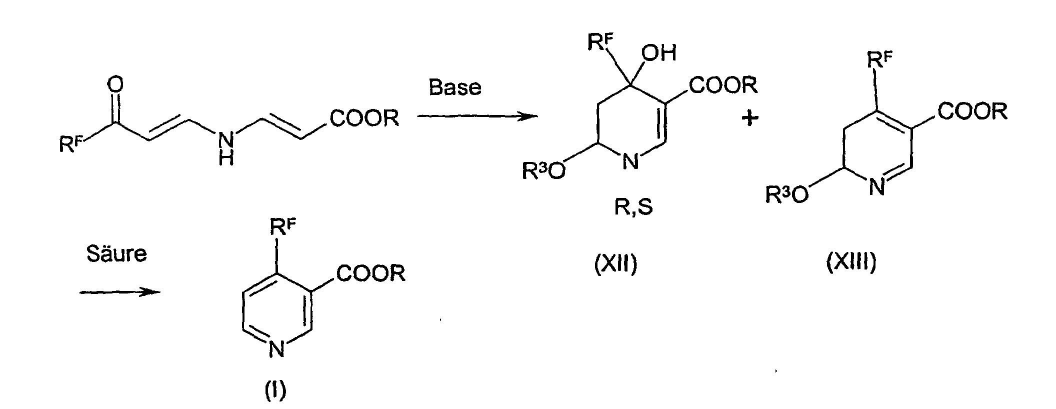

Dabei bedeutet R³ einen, vorzugsweise geradkettigen (C₁-C₆)-, vorzugsweise (C₁-C₄)- insbesondere (C₁-C₂)-, Alkylrest.

Als Base eignen sich beispielsweise Alkalimetallcarbonate, -hydrogencarbonate und -acetate, wie die entsprechenden Li, Na, K und Cs-Salze, Erdalkalimetallcarbonate und -hydrogencarbonate, wie die entsprechenden Mg- und Ca-Salze.

Bevorzugt sind Alkali- und Erdalkalicarbonate, -Hydrogencarbonate und Acetate, wie Li₂CO₃, Na₂CO₃, NaHCO₃, K₂CO₃, CaCO₃ und MgCO₃. Besonders bevorzugt sind Li₂CO₃, Na₂CO₃ und K₂CO₃, ganz besonders bevorzugt Li₂CO₃ und K₂CO₃. durch die beiden letztgenannten Basen läßt sich insbesondere die Selektivität der Reaktion in Richtung des gewünschten Endprodukts (I) erhöhen und eine Verseifung von Ester vermeiden.

Die Basen können einzeln oder in Mischung eingesetzt werden. Im allgemeinen werden 0,05 bis 1 Äquivalent, vorzugsweise 0,1 bis 0,8 Äquivalent Base pro Mol Verbindung der Formel (VI) und/oder (VIII) eingesetzt. Die Base kann gegebenenfalls nach der Reaktion abfiltriert und wieder eingesetzt werden.

Die Aktivität und Selektivität der Base können durch Phasentransferkatalysatoren (PTK) gesteuert werden. Als PTK eignen sich typischerweise Kronenether, Kryptanden, quartäre Ammonium-, Phosphonium- und Onium-Verbindungen. Beispielhaft seien genannt 12-Krone-4, 15-Krone-5, 18-Krone-6, Dibenzo-18-Krone-6, Dicyclohexyl-18-Krone-6, Tetrabutylammoniumchlorid und -bromid, Tetrabutylphosphoniumchlorid und -bromid. Bevorzugt ist 18-Krone-6. Der PTK wird üblicherweise in einer Menge von 1 bis 10, vorzugsweise 1 bis 5 Mol%, bezogen auf die Verbindung (VI) und/oder (VII), eingesetzt.

Die Zwischenprodukte der Formel (XII) und/oder (XIII) können nach üblichen, dem Fachmann geläufigen Methoden isoliert werden, beispielsweise durch Entfernen des Lösungsmittels und Auswaschen des Rückstands.

Bevorzugt ist es jedoch, die Zwischenprodukte der Formel (XII) und/oder (XIII) ohne vorherige Isolierung durch Behandeln mit Säure zu Verbindung (I) umzusetzen.

Bevorzugt sind dabei starke Säuren, wie wäßrige oder gasförmige HCl, HBr, H₂SO₄ und CF₃COOH. Der pH-Wert der Reaktionsmischung wird im allgemeinen auf 1 bis 2 eingestellt, was üblicherweise durch Verwendung von 0,1 bis 1 Äquivalenten Säure, bezogen auf die theoretische Menge an Verbindung (I), erreicht wird.

Die Umsetzung von Ester (I) zum Säureamid (VIII) kann nach bekannten, dem Fachmann geläufigen Methoden erfolgen, wie sie beispielsweise in Houben Weyl, Methoden der organischen Chemie beschrieben sind.

In einer weiteren, bevorzugten Varianten des erfindungsgemäßen Verfahrens wird die Synthese der Verbindungen (I) und (VIII) in einer Eintopfreaktion durchgeführt, d.h. ohne daß Zwischenprodukte der Formeln (V), (VI) und/oder (XII)/(XIII) isoliert werden.

Die Verbindungen (I) und (VIII) finden z.B. als Zwischenprodukte bei der Herstellung von Pflanzenschutzmitteln, insbesondere Schädlingsbekämpfungsmitteln, wie Insektiziden, Verwendung.

Insbesondere eignen sie sich zur weiteren Umsetzung zu Verbindungen wie sie in der WO-A 98/57969, EP-A 0 580 374 und der DE 100 14 006.8 beschrieben sind. Auf diese Dokumente, insbesondere die Verbindungen der jeweiligen Formel (I) und die Ausführungsbeispiele wird hiermit ausdrücklich Bezug genommen;

Gegenstand der Erfindung ist auch ein Verfahren zur Herstellung von insektizid wirksamen 4-Haloalkylnicotinsäureamiden gemäß WO-A 98/57969, EP-A 0 580 374 und/oder der DE 100 14 006.8, wobei man wie oben beschrieben 4-Haloalkylnicotinsäureester herstellt, gegebenenfalls verseift und nach in den zitierten Dokumenten beschriebenen Verfahren weiter zu den insektizid wirksamen Endverbindungen der jeweiligen Formel (I) umsetzt.

Auf den Inhalt der deutschen Patent DE 10144410, deren Priorität die vorliegende Anmeldung beansprucht, sowie der beiliegenden Zusammenfassung wird ausdrücklich Bezug genommen;

Die Erfindung wird durch die folgenden Beispiele weiter erläutert, ohne sie dadurch einzuschränken.

### Beispiel 1

### Isomerengemisch von 3-(4,4,4-Trifluor-3-oxo-1-butenylamino)-2-methylacrylat

In einem 1 I Vierhalskolben mit Thermometer, KPG Rührer, Tropftrichter mit Blasenzähler, absteigendem Kühler mit gekühlter (-10°C) Vorlage und Vakuumanschluß wurden unter N₂ 117 g Kalium-tert-butylat in 700 ml DMF vorgelegt und die Lösung auf 0°C gekühlt. 142 g 4-Amino-1,1,1-trifluor-3-buten-2-on wurde bei dieser Temperatur innerhalb von 30 min zugetropft. Nach beendeter Zugabe wurde die Lösung noch 30 min nachgerührt, und dann wurden bei dieser Temperatur 148 g 3,3-Dimethoxypropionsäuremethylester zugetropft. Der Tropftrichter wurde entfernt und der Druck im System wurde langsam auf 20-25 mbar reduziert.

Anschließend wurde das Gemisch 3-5 h bei 30-35 °C und 20-25 mbar Vakuum gerührt, wobei die leichtsiedenden Produkte (Methanol, tert. Butanol) gleichzeitig im Vakuum entfernt und in der Vorlage kondensiert wurden.

Das Reaktionsgemisch wurde auf 1000 g Eis mit 40 ml HCl (d 1.19) bei 0-10 °C gegeben und mit HCl auf pH 2-3 gestellt. Nach 1 h wurde der Niederschlag abfiltriert, mit 300 ml Eiswasser gewaschen und das Produkt getrocknet. Man erhielt 205 g (92 %) von 3-(4,4,4-Trifluor-3-oxo-1-butenylamino)methylacrylat als Isomerengemisch von 2 Stereoisomeren.

### Beispiel 2

### 4-Trifluormethyl-3-pyridincarbonsäuremethylester

In einem Dreihalskolben wurden 19 g (0,1 mol) 3-(4,4,4-Trifluor-3-oxo-1-butenylamino)-methylacrylat in 200 ml Methanol gelöst, und 1 g Li₂CO₃ wurde zugegeben. Das Reaktionsgemisch wurde für 6-8 h unter Rückfluß erhitzt, auf 30°C gekühlt und 10 ml wäßrige HCl wurden zugegeben. Das Reaktionsgemisch wurde 1 h gerührt, das Methanol im Vakuum entfernt und das Produkt mit Diethylether extrahiert. Das Lösemittel wurde entfernt und 4-Trifluomicotinsäuremethylester durch Vakuumdestillation gereinigt. Man erhielt 14 g (81 %) des Produktes mit S.p 80 °C/18 mbar.
NMR ¹H (CDCl₃) δ : 9.17 (s, 1 H), 8.97 (d, 1H, ³J_{(H,H)} = 5 Hz), 7.71 (d,1H,), 4.04 (s,3H) ppm.
NMR ¹⁹F δ : -62.4 (s, CF₃) ppm.

### Beispiel 3

### 4-Trifluormethyl-3-pyridincarbonsäuremethylester

Man arbeitete wie in Beispiel 2 beschrieben, anstelle von Li₂CO₃ nahm man jedoch 1 g K₂CO₃. Ausbeute 75%.

### Beispiel 4

### 4-Trifluormethyl-3-pyridincarbonsäuremethylester

Man arbeitete wie in Beispiel 2 beschrieben, anstelle von Li₂CO₃ nahm man jedoch 1 g Natriumacetat. Ausbeute 68 %.

### Beispiel 5

### 4-Hydroxy-6-methoxy-4-(trifluormethyl)-1,4,5,6-tetrahydro-3-methylpyridincarboxylat. (R und S) isomer.

In einem Dreihalskolben wurden unter N₂ 19 g (0,01 mol) 3-(4,4,4-Trifluor-3-oxo-1-butenylamino)-2-methylacrylat in 200 ml Methanol gelöst und 2 g Li₂CO₃ wurden zugegeben. Das Reaktionsgemisch wurde für 8 h bei 50-60 C gerührt, der Niederschlag wurde abfiltriert und das Methanol weitgehend in Vakuum entfernt. Die beide Isomere wurden durch Chromatografie auf SiO₂ (Laufmittel Ethylacetat/Heptan): getrennt und gereinigt. Ausbeute 88%.
Isomer 1. Ausbeute 44%. M.P. 148-149°C.
1H NMR (CDCl3) (ABX Spin System) δ: 1.98 dm (H_{A}), ²J(_{HaHb})= 17 Hz; 2.68 dm (H_{B}), 3.39 (s,3H, OCH₃), 3.73 (s,3H, OCH₃), 4.57 m (1H), 5.6 bs (NH); 6,33 (1H, OH); 7.6 d (1H,CH, ³J =7 Hz) ppm.
NMR ¹⁹F δ: -79.3 (s) ppm.
Isomer 2. Ausbeute 43% (viskose Öl)
1H NMR (CDCl3) (ABX Spin System) δ: 1.97 ddkw (H_{A}), ²J(_{HaHb})= 16 Hz; 2.53 ddd (H_{B}) ³J =5.2, ³J=1.5 Hz, 3.42 (s,3H, OCH₃), 3.71 (s,3H, OCH₃), 4.57 dd (1H), 5.6 bs (NH); 6,6 (1H, OH); 7.6 d (1H,CH, ³J =7 Hz) ppm.
NMR ¹⁹F δ: -81.0 (s) ppm.

Die beide Produkte reagieren mit HCl bei RT zu 4-Trifluormethyl-3-methylpyridincarboxylat. Ausbeute 95%.

### Beispiel 6 (Vergleichsbeispiel)

### 3-(4,4,4-Trifluor-3-oxo-1-butenylamino)methylacrylat

Man arbeitete wie in Beispiel 1 beschrieben jedoch ohne vermindertem Druck.
Ausbeute 77%, Reinheit 95%.

### Beispiel 7

### 3-(4,4,4-Trifluor-3-oxo-1-butenylamino)methylacrylat

Man arbeitete wie in Beispiel 1 beschrieben, nahm jedoch statt 3,3-Dimethoxypropionsäuremethylester, 3-Methoxyacrylsäuremethylester.
Ausbeute 91%

## Patentansprüche

1. Verfahren zur Herstellung von 4-Haloalkylnicotinsäureestern der Formel (I), 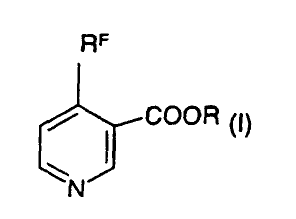 worin
R^{F} (C₁-C₄)-Haloalkyl bedeutet und
R (C₁-C₆)-Alkyl bedeutet;
wobei man
a) ein Aminoketon der Formel (II),
R^{F}-C(O)-CH=CH-NH₂ (II)
in einer Kondensationsreaktion under einem verminderten Druck im Bereich von 5 bis 150 mbar mit einer Verbindung der Formel (III) bis (V),
(R¹Z)CH=CH-COOR (III),
(R¹Z)₂CH-CH₂-COOR (IV),
HC≡ C-COOR (V),
wobei
R¹ (C₁-C₆)-Alkyl und
Z O, S, NR¹ oder C(O)O bedeutet,
zu einer Verbindung der Formel (VI) und/oder (VII) umsetzt,
R^{F}-C(O)-CH=CH-NH-CH=CH-COOR (VI)
R^{F}-C(O)-CH=CH-NH-CH(ZR¹)-CH₂-COOR (VII)
b) und das Reaktionsprodukt einer Ringschlußreaktion unterwirft.

2. Verfahren nach Anspruch 1, wobei in den Formel (I) - (VII)
R CH₃ und
R^{F} CF₃ bedeutet.

3. Verfahren nach einem oder mehreren der vorliegenden Ansprüche, wobei man Schritt a) in Gegenwart einer Base aus der Gruppe KO-^{t}Bu und NaOMe durchführt.

4. Verfahren nach einem oder mehreren der vorherigen Ansprüche, wobei man Schritt b) in Gegenwart eines Alkali- und/oder Erdalkalicarbonats durchführt.

5. Verfahren zur Herstellung von 4-(C₁-C₄)-Haloalkyl-nicotinsäureamiden (VIII), 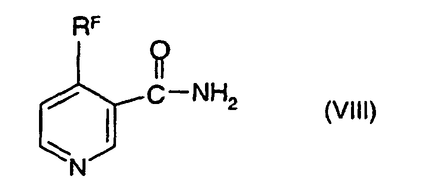 worin
R^{F} (C₁-C₄)-Haloalkyl bedeutet,
wobei man einen gemäß dem Prozeß nach einem oder mehreren der Ansprüche 1 bis 5 erhaltenen 4-(C₁-C₄)-Haloalkylnicotinsäureester der Formel (I), 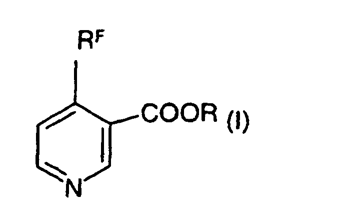 worin
R^{F} (C₁-C₄)-Haloalkyl und
R (C₁-C₆)-Alkyl bedeutet;
einer Ammonolyse unterwirft.

6. Verfahren zur Herstellung von Verbindungen der Formel (VI) und/oder (VII),
R^{F}-C(O)-CH=CH-NH-CH=CH-COOR (VI)
R^{F}-C(O)-CH=CH-NH-CH(ZR¹)-CH₂-COOR (VII)
worin
R^{F} (C₁-C₄)-Haloalkyl,
R, R¹ gleich oder verschieden (C₁-C₆)-Alkyl und
Z O, S, NR¹ oder C(O)O bedeuten,
wobei
ein 4-Aminobutenon der Formel (II),
R^{F}-C(O)-CH=CH-NH₂ (II)
in einer Kondensationsreaktion unter einem verminderten Druck im Bereich von 5 bis 150 mbar mit einer Verbindung der Formel (III) bis (V),
(R¹Z)CH=CH-COOR (III)
(R¹Z)₂CH-CH₂-COOR (IV)
HC≡C-COOR (V)
umsetzt.

## Claims

1. A process for preparing 4-haloalkylnicotic esters of the formula (I), 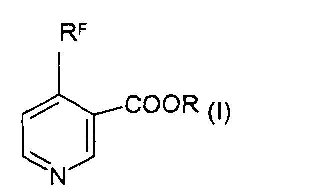 where
R^{F} is (C₁-C₄)-haloalkyl and
R is (C₁-C₆)-alkyl;
which comprises
a) reacting an aminoketone of the formula (II),
R^{F}-C(O)-CH=CH-NH₂ (II)
in a condensation reaction under a reduced pressure in the range from 5 to 150 mbar with a compound of the formulae (III) to (V),
(R¹Z)CH=CH-COOR (III),
(R¹Z)₂CH-CH₂-COOR (IV),
HC≡C-COOR (V),
where
R¹ is (C₁-C₆)-alkyl and
Z is O, S, NR¹ or C(O)O,
to give a compound of the formulae (VI) and/or (VII),
R^{F}-C(O)-CH=CH-NH-CH=CH-COOR (VI)
R^{F}-C(O)-CH=CH-NH-CH(ZR¹)-CH₂-COOR (VII)
b) and subjecting the reaction product to a ring closure reaction.

2. The process as claimed in claim 1, wherein, in the formulae (I) - (VII),
R is CH₃ and
R^{F} is CF₃.

3. The process as claimed in one or more of the preceding claims, wherein step a) is carried out in the presence of a base selected from the group consisting of KO-^{t}Bu and NaOMe.

4. The process as claimed in one or more of preceding claims, wherein step b) is carried out in the presence of an alkali metal carbonate and/or alkaline earth metal carbonate.

5. A process for preparing 4-(C₁-C₄)-haloalkylnicotinamides (VIII), 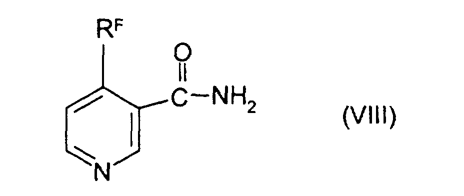 where
R^{F} is (C₁-C₄)-haloalkyl,
which comprises subjecting a 4-(C₁-C₄)-haloalkylnicotinic ester of the formula (I) obtained according to the process as claimed in one or more of claims 1 to 5, 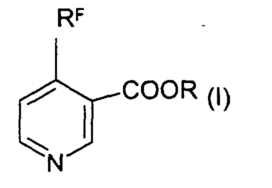 where
R^{F} is (C₁-C₄)-haloalkyl and
R is (C₁-C₆)-alkyl;
to an ammonolysis.

6. A process for preparing compounds of the formulae (VI) and/or (VII),
R^{F}-C(O)-CH=CH-NH-CH=CH-COOR (VI)
R^{F}-C(O)-CH=CH-NH-CH(ZR¹)-CH₂-COOR (VII)
where
R^{F} is (C₁-C₄)-haloalkyl,
R, R¹ are identical or different (C₁-C₆)-alkyl and
Z is O, S, NR¹ or C(O)O,
which comprises
reacting a 4-aminobutenone of the formula (II),
R^{F}-C(O)-CH=CH-NH₂ (II)
in a condensation reaction under a reduced pressure in the range from 5 to 150 mbar with a compound of the formulae (III) to (V),
(R¹Z)CH=CH-COOR (III)
(R¹Z)₂CH-CH₂-COOR (IV)
HC≡C-COOR (V).

## Revendications

1. Procédé pour la préparation d'esters de l'acide 4-haloalkylnicotinique de formule (I) 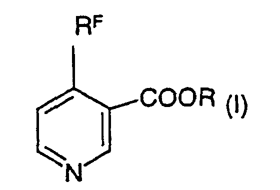 où
R^{F} représente un groupe haloalkyle en C₁-C₄ et
R représente un groupe alkyle en C₁-C₆ ;
a) en faisant réagir une aminocétone de formule (II),
**R**^{**F**}**-C(O)-CH=CH-NH**_{**2**} **(II)**
dans une réaction de condensation sous pression réduite dans le domaine de 5 à 150 mbars sur un composé de formule (III) à (V)
**(R**^{**1**}**Z)CH=CH-COOR (III),**
**(R**^{**1**}**Z)**_{**2**}**CH-CH**_{**2**}**-COOR (IV),**
**HC≡ C-COOR (V),**
dans lesquelles
R¹ représente un groupe alkyle en C₁-C₆ et
Z représente un groupe O, S, NR¹ ou C(O)O,
en vue d'obtenir un composé de formule (VI) et/ou (VII)
**R**^{**F**}**-C(O)-CH=CH-NH-CH=CH-COOR (VI)**
**R**^{**F**}**-C(O)-CH=CH-NH-CH(ZR**^{**1**}**)-CH**_{**2**}**-COOR (VII)**
b) et en soumettant le produit réactionnel à une réaction de cyclisation.

2. Procédé selon la revendication 1, dans les formules (I) à (VII)
R représente un groupe CH₃ et
R^{F} représente un groupe CF₃.

3. Procédé selon une ou plusieurs des revendications présentes, dans l'étape a) étant mise en oeuvre en présence d'une base prise dans le groupe comprenant KO-^{t}Bu et NaOMe.

4. Procédé selon une ou plusieurs des revendications précédentes, l'étape b) étant mise en oeuvre en présence d'un carbonate alcalin et/ou alcalino-terreux.

5. Procédé pour la préparation d'amides de l'acide 4-halo(alkyl en C₁-C₄)nicotinique (VIII) 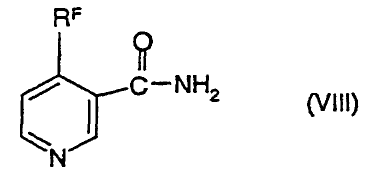 où
R^{F} représente un groupe haloalkyle en C₁-C₄,
dans lequel un ester de l'acide 4-halo(alkyl en C₁-C₄)nicotinique, obtenu selon le procédé selon une ou plusieurs des revendications 1 à 5, de formule (I) 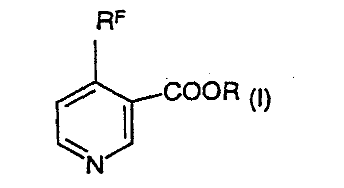 où
R^{F} représente un groupe haloalkyle en C₁-C₄ et
R représente un groupe alkyle en C₁-C₆,
est soumis à une ammonolyse.

6. Procédé pour la préparation de composés de formule (VI) et/ou (VII),
**R**^{**F**}**-C(O)-CH=CH-NH-CH=CH-COOR (VI)**
**R**^{**F**}**-C(O)-CH=CH-NH-CH(ZR**^{**1**}**)-CH**_{**2**}**-COOR (VII)**
où
R^{F} représente un groupe haloalkyle en C₁-C₄,
R, R¹ identiques ou différents représentent un groupe alkyle en C₁-C₆ et
Z représente un groupe O, S, NR¹ ou C(O)O,
en faisant réagir une 4-aminobuténone de formule (II)
**R**^{**F**}**-C(O)-CH=CH-NH**_{**2**} **(II)**
dans une réaction de condensation sous une pression réduite dans le domaine de 5 à 150 mbars, sur un composé de formule (III) à (V),
**(R**^{**1**}**Z)CH=CH-COOR (III),**
**(R**^{**1**}**Z)**_{**2**}**CH-CH**_{**2**}**-COOR (IV),**
**HC≡ C-COOR (V),**
.
